# EUROPEAN PATENT APPLICATION

(11) **EP 4 385 425 A1**
(43) Date of publication of application: **19.06.2024**
(21) Application number: 22855402.8
(22) Date of filing: 08.08.2022
(51) Int. Cl.: A61B 17/00, A61B 17/34

(54) **MEDICAL STENT OPERATING SYSTEM**

(30) Priority: 08.08.2021 US 202163230759 P
(71) Applicant: Chen, Chieh-Hsiao, Taichung City, Taiwan 406 (CN)
(72) Inventor: Chen, Chieh-Hsiao, Taichung City, Taiwan 406 (CN)
(74) Representative: Lucke, Andreas
(86) International application number: PCT/CN2022/110924
(87) International publication number: WO 2023/016421

(57) **Abstract**

A medical stent operating system includes: a tube sheath portion, having a first cavity extending along a length direction of the tube sheath portion; a holding portion, connected to the tube sheath portion, and having a second cavity; an operating portion, movably connected to the holding portion, the operating portion having a first operating end and a second operating end, the first operating end disposed in the second cavity, the second operating portion disposed outside the holding portion; and a stent movement portion, connected to the first operating end and extending into the first cavity and the second cavity, wherein an end of the stent movement portion comes into contact with a medical stent; wherein the operating portion moves relative to the holding portion, and moves the medical stent via the stent movement portion.

## Description

### BACKGROUND OF THE INVENTION

### FIELD OF THE INVENTION

The present invention relates to a medical stent operating system, and more particularly, to a medical stent operating system that helps a medical stent be placed into a human body and assist the medical stent be removed from a human body.

### DESCRIPTION OF THE PRIOR ART

Conventional medical stents used for prostate surgery or bladder neck incision surgery can improve the problem of prostate hypertrophy. However, how to place a medical stent to a correct location in a human body or how to remove a medical stent from a human body needs to be performed by doctors based on their experience, and this often involves certain risks. In view of the above, there is a need for a medical stent operating system that helps a medical stent be placed into a human body and assist the medical stent be removed from a human body.

### SUMMARY OF THE INVENTION

To overcome the above issues, it is a concept of the present invention to provide a medical stent operating system that helps a medical stent be placed into a human body and assist the medical stent be removed from a human body.

On the basis of the concept above, the present invention provides a medical stent operating system including: a tube sheath portion, having a first cavity extending along a length direction of the tube sheath portion; a holding portion, connected to the tube sheath portion, and having a second cavity; an operating portion, movably connected to the holding portion, the operating portion having a first operating end and a second operating end, the first operating end disposed in the second cavity, the second operating portion disposed outside the holding portion; and a stent movement portion, connected to the first operating end and extending into the first cavity and the second cavity, wherein an end of the stent movement portion comes into contact with a medical stent; wherein the operating portion moves relative to the holding portion, and moves the medical stent via the stent movement portion.

In a preferred embodiment of the present invention, the tube sheath portion appears as a transparent tube.

In a preferred embodiment of the present invention, the medical stent operating system further includes: an optical sensing device, including a sensing unit and a transmission unit. The sensing unit is connected to the transmission unit, and is disposed on a holding end of the holding portion. Moreover, the sensing unit is at least partially disposed in the tube sheath portion, and is located on a tube sheath end of the tube sheath portion. The transmission unit at least partially extends into the first cavity.

In a preferred embodiment of the present invention, the stent movement portion includes a pushing unit disposed on an end of the stent movement portion. The pushing unit is shaped as a hollow cylinder, and sleeves the transmission unit in a manner of being movable relative to the transmission unit.

In a preferred embodiment of the present invention, the tube sheath portion includes a tube sheath end having a lateral communication outlet.

In a preferred embodiment of the present invention, the stent movement portion includes a hook unit disposed on an end of the stent movement portion.

In a preferred embodiment of the present invention, the tube sheath portion is detachably connected to the holding portion.

In a preferred embodiment of the present invention, the stent movement portion includes a pushing unit disposed on an end of the stent movement portion.

In a preferred embodiment of the present invention, the medical stent has a first expansion section, a second expansion section and a third expansion section, wherein the third expansion section is disposed between the first expansion section and the second expansion section. The medical stent operating system includes a staged movement mechanism. The operating portion moves in stages relative to the holding portion via the staged movement mechanism, and sequentially pushes the first expansion section, the third expansion section and the second expansion section in stages out of the tube sheath portion by the stent movement portion.

In a preferred embodiment of the present invention, the staged movement mechanism is the holding portion having a plurality of fastening holes. The first operating end of the operating portion includes a fastening unit, which fastens with one of the plurality of fastening holes during a process of the operating portion moving relative to the holding portion.

In a preferred embodiment of the present invention, the plurality of fastening holes include a first fastening hole, a second fastening hole and a third fastening hole. When the fastening unit is fastened with the first fastening hole, the first expansion section is pushed out of the tube sheath portion by the stent movement portion. When the fastening unit is fastened with the second fastening hole, the third expansion section is pushed out of the tube sheath portion by the stent movement portion. When the fastening unit is fastened with the third fastening hole, the second expansion section is pushed out of the tube sheath portion by the stent movement portion.

In a preferred embodiment of the present invention, the holding portion has an elongated through hole extendedly disposed on the holding portion along a length direction of the holding portion. The first operating end of the operating portion includes a protruding unit, which protrudes outward through the elongated through hole and is movable along the elongated through hole.

In a preferred embodiment of the present invention, the medical stent has a first expansion section, a second expansion section and a third expansion section, wherein the third expansion section is disposed between the first expansion section and the second expansion section. The elongated through holes has a through hole end. The medical stent operating system causes the protruding unit to come into contact with the through hole end, such that the operating portion is unable to continue moving relative to the holding portion along a push-out direction. When the protruding unit is in contact with the through hole end, the medical stent operating system causes the second expansion section of the medical stent to be partially exposed outside the tube sheath portion via the stent movement portion.

In a preferred embodiment of the present invention, the protruding unit receives a first external force, departs from the through hole end and departs from the elongated through hole. The operating portion next receives a second external force and moves relative to the holding portion along the push-out direction, and causes the second expansion section to be pushed out of the tube sheath portion by the stent movement portion.

In a preferred embodiment of the present invention, the tube sheath portion includes a liquid transport unit in communication with the first cavity. The medical stent operating system transports a liquid into the tube sheath portion via the liquid transport unit.

To provide better understanding, the above and other aspects of the present invention are to be described with reference by way of the non-limiting embodiments and the accompanying drawings below.

### BRIEF DESCRIPTION OF THE DRAWINGS

FIG. 1 is a schematic diagram of a medical stent operating system according to a specific embodiment of the present invention;
FIG. 2A is a schematic diagram of a holding portion and an optical sensing device of a medical stent operating system according to a specific embodiment of the present invention;
FIG. 2B is a schematic diagram of an operating portion and a stent movement portion of a medical stent operating system according to a specific embodiment of the present invention;
FIG. 2C is a schematic diagram of a tube sheath portion medical stent operating system according to a specific embodiment of the present invention;
FIG. 3 is a partial schematic diagram of a stent movement portion of a medical stent operating system according to a specific embodiment of the present invention;
FIG. 4 is a partial schematic diagram of a tube sheath portion, an extension unit and a pushing unit according to a specific embodiment; and
FIG. 5 is a partial schematic diagram of a tube sheath portion, an extension unit and a hook unit according to a specific embodiment.

### DETAILED DESCRIPTION OF THE EMBODIMENTS

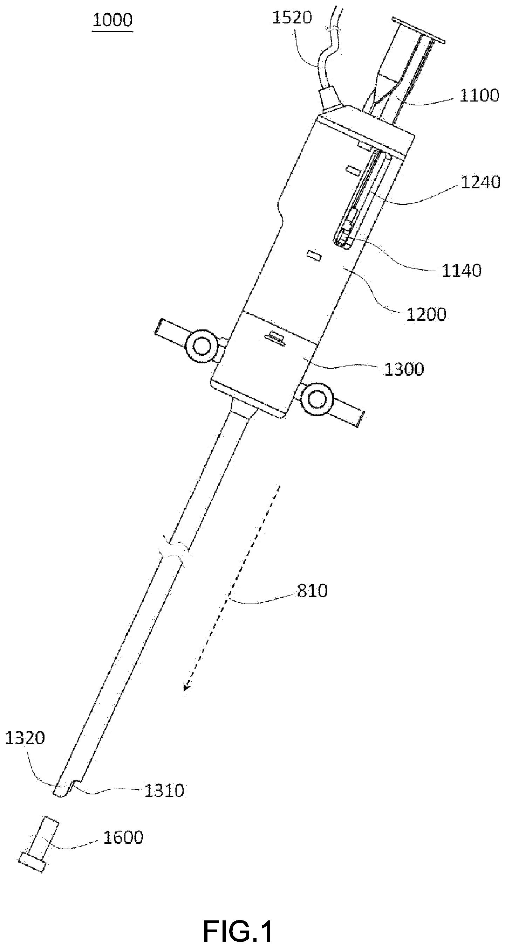

Refer to FIG. 1 showing a schematic diagram of an example of a medical stent operating system according to a specific embodiment of the present invention. As shown by the embodiment in FIG. 1, the medical stent operating system 1000 includes an operating portion 1100, a holding portion 1200, a tube sheath portion 1300, a stent movement portion (not shown, to be described in detail later), an optical sensing device ((not shown, to be described in detail later), and a protection portion 1600. The tube sheath portion 1300 is a hollow element having a first cavity therein, wherein the first cavity extends along a length direction 810 of the tube sheath portion 1300. Preferably, a tube sheath end 1320 of the tube sheath portion 1300 can have a lateral communication outlet 1310 (configuration details of the lateral communication outlet 1310 can be referred from Taiwan Patent Application No. 110103890, entitled "SURGICAL-USE MEDICAL APPARATUS AND SYSTEM THEREFOR". The full text of the above Taiwan Patent Application No. 110103890 is incorporated herein by reference). The holding portion 1200 is a hollow element having a second cavity therein. The tube sheath portion 1300 is detachably connected to the holding portion 1200. The operating portion 1100 is movably connected to the holding portion 1200. The operating portion 1100 includes a first operating end and a second operating end. The first operating end of the operating portion 1100 is disposed in the second cavity of the holding portion 1200, and the second operating end of the operating portion 1100 is disposed outside the holding portion 1200. Preferably, a connection relationship and an operation relationship between the operating portion 1100 and the holding portion 1200 are similar to a push rod and a syringe of a common medical syringe device.

The stent movement portion of the medical stent operating system 1000 is connected to the first operating end of the operating portion 1100, and the stent movement portion extends into the first cavity and the second cavity. During a process of operating (or moving) a medical stent by the medical stent operating system 1000, the medical stent can be first placed in the tube sheath portion 1300 (preferably, the medical stent is first placed in the tube sheath portion 1300, and is located at a position near the tube sheath end 1320 of the tube sheath portion 1300), and the medical stent is made to come into contact with an end of the stent movement portion. By moving the operating portion 1100 relative to the holding portion 1200, a user can move the medical stent via the stent movement portion connected to the operating portion 1100, and gradually push the medical stent out of the tube sheath portion 1300. The protection portion 1600 is detachably installed (for example, plugged) at the tube sheath end 1320 of the tube sheath portion 1300, so as to protect the tube sheath portion 1300 and the stent movement portion in the tube sheath portion 1300 and the optical sensing device. A transmission unit 1520 (for example, a transmission line) of the optical sensing device can be extendedly disposed at the end proximate to operating portion 1100, of the holding portion 1200. It should be understood that, during a process of using the medical stent operating system 1000, the protection portion 1600 is not needed and can be removed from the tube sheath end 1320 of the tube sheath portion 1300. In a specific embodiment, the protection portion 1600 is not necessary.

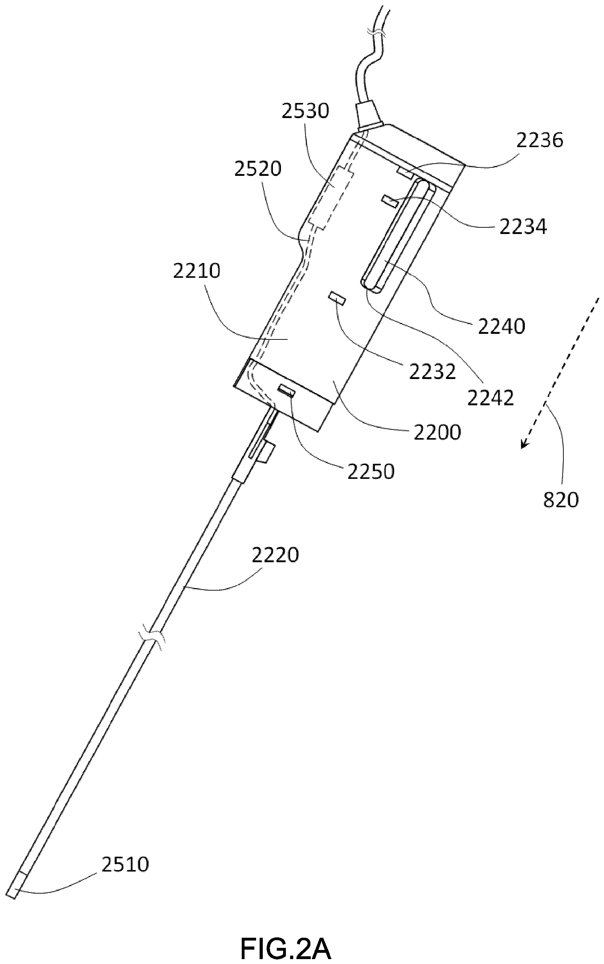

Refer to FIG. 2A showing a schematic diagram of examples of a holding portion and an optical sensing device of a medical stent operating system according to a specific embodiment of the present invention. As shown by the embodiment in FIG. 2A, a holding portion 2200 includes a holding body 2210 and an extension unit (or can be referred to as a holding extension unit) 2220, wherein the holding body 2210 is connected to the extension unit 2220. The holding body 2210 has fastening holes (which can be referred to as holding fastening holes) 2232, 2234 and 2236, an elongated through hole 2240 and a holding fastening unit 2250. The fastening hole 2232 is closer to the extension unit 2220 than the elongated through hole 2240, and the elongated through hole 2240 is extendedly disposed on the holding body 2210 of the holding portion 2200 along a length direction of the holding portion 2200. Preferably, a through hold end 2242 of the elongated through hole 2240 is disposed between the fastening hole 2234 and the fastening hole 2232. The optical sensing device of the medical stent operating system includes a sensing unit 2510 and a transmission unit 2520, wherein the sensing unit 2510 is connected to the transmission unit 2520. The sensing unit 2510 is disposed (or connected) on an end (or referred to as a holding end) of the extension unit 2220. The transmission unit 2520 is extendedly disposed in the second cavity of the holding portion 2200 (more specifically, the transmission unit 2520 is extendedly disposed in the extension unit 2220 and in the holding body 2210, and extends outward from the extension body 2210). The sensing unit 2510 can capture an in-vivo image during an operation process of the medical stent operating system, and transmit the captured image by the transmission unit to a display device for a user to view the image. In a specific embodiment, the transmission unit 2520 can include a processing unit 2530 capable of assisting in processing the image captured by the sensing unit 2510.

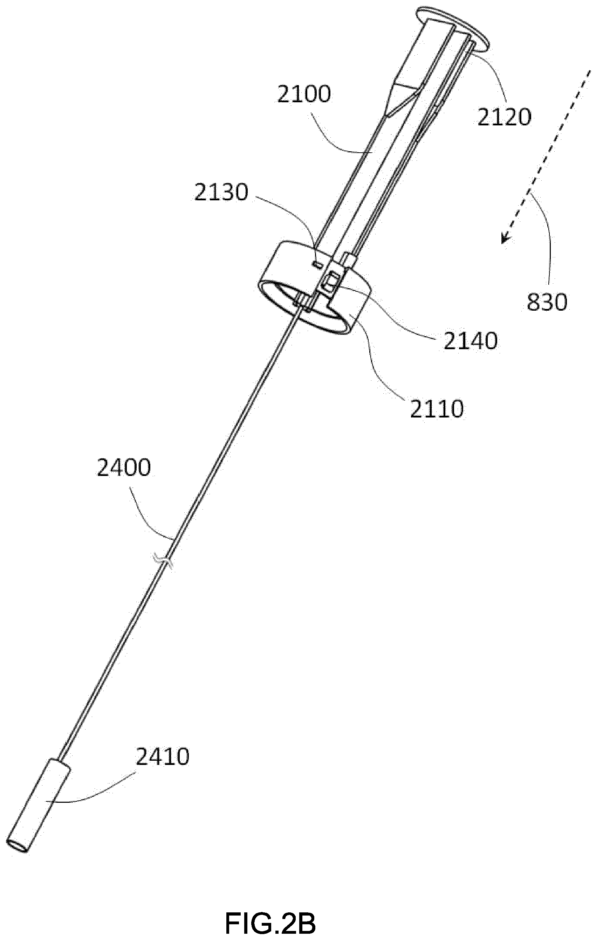

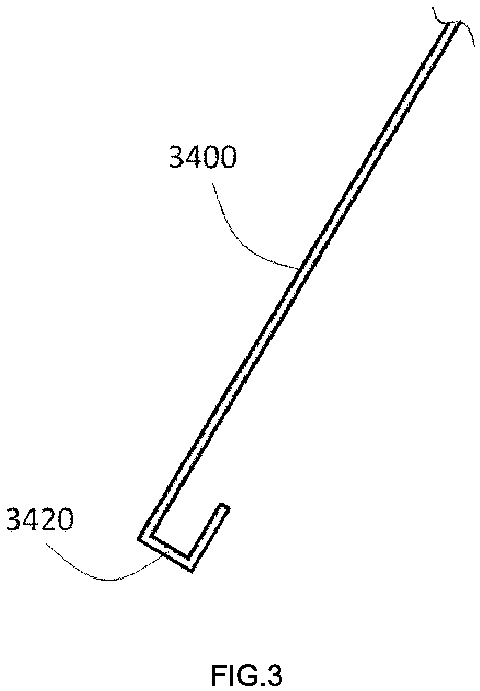

Refer to FIG. 2B showing a schematic diagram of examples of an operating portion and a stent movement portion of a medical stent operating system according to a specific embodiment of the present invention. As shown by the embodiment in FIG. 2B, the operating portion 2100 includes a first operating end 2110, a second operating end 2120, a fastening unit 2130 and a protruding unit 2140. The fastening unit 2130 and the protruding unit 2140 are both disposed on the first operating end 2110. One end of the stent movement portion 2400 is connected to the operating portion 2100, and the other end of the stent movement portion 2400 (or referred to as an end or a stent movement end) includes a pushing unit 2410. Thus, when a user moves (or operates) the operating portion 2100, the stent movement portion 2400 moves along with the operating portion 2100, thereby pushing the medical stent by the pushing unit 2410. In a specific embodiment, the end of the stent movement portion 2400 does not include the pushing unit but includes a hook unit. Thus, a user can hook the medical stent by the hook unit, and then pull the medical stent by moving (or operating) the operating portion 2100. FIG. 3 shows an example of an end of the stent movement portion 2400 having a hook unit according to a specific embodiment. As shown by the embodiment in FIG. 3, the end of a stent movement portion 3400 includes a hook unit 3420.

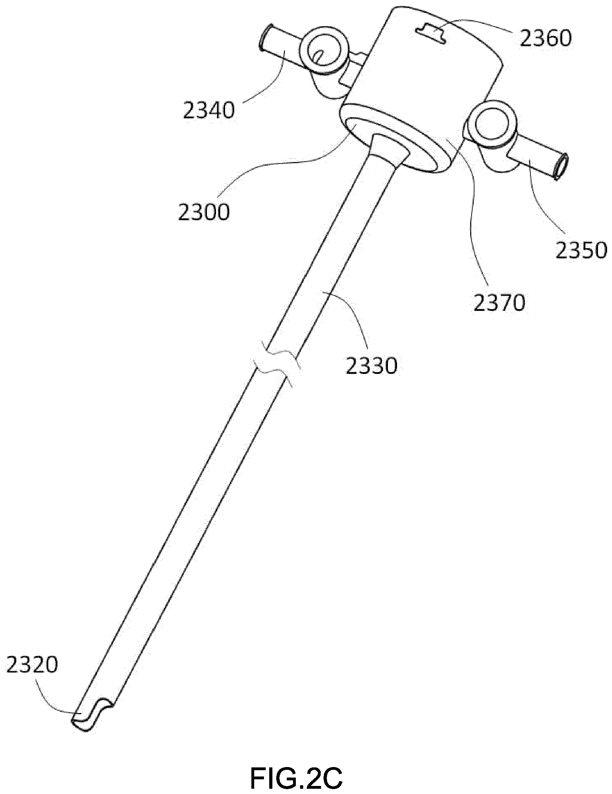

Refer to FIG. 2C showing a schematic diagram of an example of a tube sheath portion of a medical stent operating system according to a specific embodiment of the present invention. As shown by the embodiment in FIG. 2C, a tube sheath portion 2300 includes a tube sheath body 2370, a tube sheath extension unit 2330, liquid transport units 2340 and 2350, and a fastening hole (or referred to as a tube sheath fastening hole) 2360. The tube sheath body 2370 is connected to the tube sheath extension unit 2330, the liquid transport units 2340 and 2350 are connected to the tube sheath body 2370, and the fastening hole 2360 is disposed on the tube sheath body 2370. The liquid transport units 2340 and 2350 are in communication with the first cavity of the tube sheath portion 2300. Thus, a liquid (for example but not limited to, water or saline) can be transported into the tube sheath portion 2300 via the liquid transport units 2340 and 2350.

Refer to FIG. 2A, FIG. 2B and FIG. 2C. The stent movement portion 2400 can be extendedly disposed in the first cavity of the tube sheath portion 2300 and in the second cavity of the holding portion 2200. The end of the stent movement portion 2400 can come into contact with the medical stent. For example, when the end of the stent movement portion 2400 includes the pushing unit 2410, the medical stent can be gradually pushed to the outside of the tube sheath portion 2300 by the pushing unit 2410 of the stent movement portion 2400. When the end of the stent movement portion 2400 includes the hook unit, the medical stent can be gradually pulled to the inside of the tube sheath portion 2300 by the hook unit of the stent movement portion 2400.

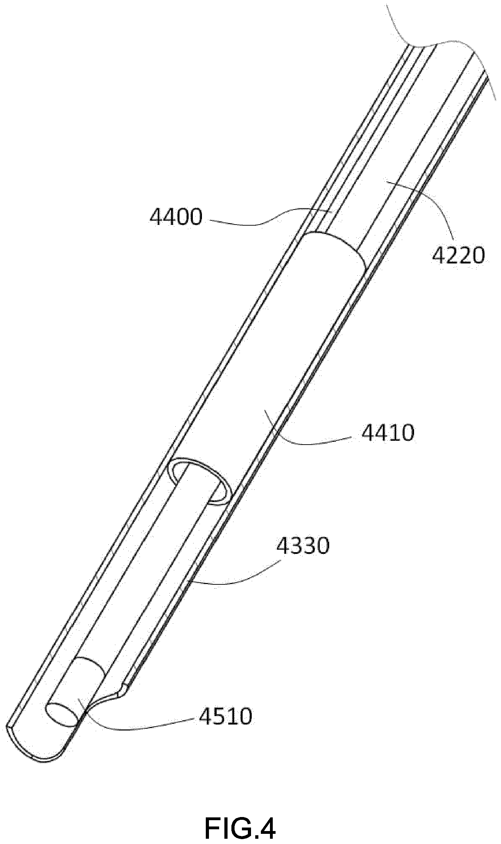

The stent movement portion 2400 and the extension unit 2220 of the holding portion 2200 can both be extendedly disposed in the first cavity of the tube sheath portion 2300. Preferably, when the end of the stent movement portion 2400 includes the pushing unit 2410, the pushing unit 2410 can be shaped as a hollow cylinder, and the pushing unit 2410 can sleeve the transmission unit 2520 and the extension unit 2220 in a manner of being movable relative to transmission unit 2520 and the extension unit 2220. FIG. 4 shows a partial schematic diagram of examples of a tube sheath portion, an extension unit and a pushing unit according to a specific embodiment. As shown by the embodiment in FIG. 4, a holding extension unit 4220 and a stent movement portion 4440 are disposed within a tube sheath extension portion 4330. An end of the stent movement portion 4400 includes a pushing unit 4410. The pushing unit 4410 is shaped as a hollow cylinder, and the pushing unit 4410 sleeves the holding extension unit 4220 in a manner of being movable relative to the holding extension unit 4220. Since the transmission unit of the optical sensing device is extendedly disposed in the holding extension unit 4220, the pushing unit 4410 also sleeves the transmission unit in a manner of being movable relative to the transmission unit. With the configuration above, when a user moves the operating portion relative to the holding portion such that the pushing unit 4410 moves relative to the holding extension unit 4220 and the transmission unit, a sensing unit 4510 located on an end of the holding extension unit 4220 can still continue capturing an image without being affected by the pushing unit 4410. Preferably, the tube sheath portion appears as a transparent tube, or at least a tube sheath extension unit 4330 of the tube sheath portion appears as a transparent tube, so that the tube sheath extension unit 4330 of the tube sheath portion can be prevented from blocking a field of view of the sensing unit 4510. In a specific embodiment, the transmission unit is partially extendedly disposed in the holding extension unit 4220 (that is, the transmission unit is partially extendedly disposed in the first cavity of the tube sheath portion), and the transmission unit is partially extendedly disposed in the second cavity of the holding portion. Preferably, during a process of image capturing of the sensing unit 4510, the sensing unit 4510 is preferably disposed in the tube sheath extension unit 4330 of the tube sheath portion, and is placed (or arranged) on a tube sheath end of the tube sheath extension unit 4330. The sensing unit 4510 can be completely arranged in the tube sheath portion or be slightly exposed outside the tube sheath portion as required. When the holding extension unit 4220 moves relative to the tube sheath extension unit 4330 (for example, when the holding extension unit 4220 is removed from the inside of the tube sheath extension unit 4330), the sensing unit 4510 can move relative to the tube sheath extension unit 4330 along with the holding extension unit 4220 (for example, the sensing unit 4510 can be removed from the inside of the tube sheath extension unit 4330 along with the holding extension unit 4220).

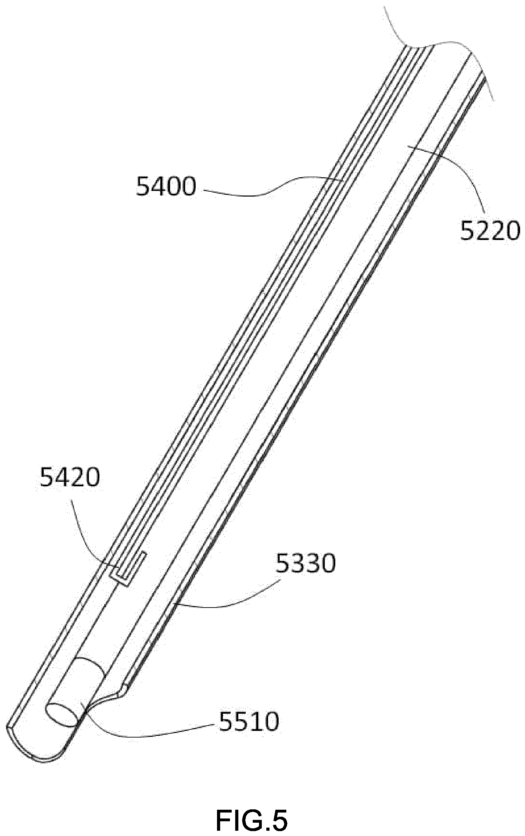

Refer to FIG. 5 showing a partial schematic diagram of examples of a tube sheath portion, an extension unit and a hook unit according to a specific embodiment. As shown by the embodiment in FIG. 5, a holding extension unit 5220 and a stent movement portion 5400 are disposed in a tube sheath extension unit 5330, and an end of the stent movement portion 5400 includes a hook unit 5420. When a user moves the operating portion relative to the holding portion such that the hook unit 5420 moves relative to the holding extension unit 5220 and the transmission unit, a sensing unit 5510 located on an end of the holding extension unit 5220 can still continue capturing an image without being affected by the hook unit 5420. Preferably, the tube sheath portion appears as a transparent tube, or at least a tube sheath extension unit 5330 of the tube sheath portion appears as a transparent tube, so that the tube sheath extension unit 5330 of the tube sheath portion can be prevented from blocking a field of view of the sensing unit 5510. Preferably, during a process of image capturing of the sensing unit 5510, the sensing unit 5510 is preferably disposed in the tube sheath extension unit 5330 of the tube sheath portion, and is placed (or arranged) on a tube sheath end of the tube sheath extension unit 5330. The sensing unit 5510 can be completely arranged in the tube sheath portion or be slightly exposed outside the tube sheath portion as required. When the holding extension unit 5220 moves relative to the tube sheath extension unit 5330 (for example, when the holding extension unit 5220 is removed from the inside of the tube sheath extension unit 5330), the sensing unit 5510 can move relative to the tube sheath extension unit 5330 along with the holding extension unit 5220 (for example, the sensing unit 5510 can be removed from the inside of the tube sheath extension unit 5330 along with the holding extension unit 5220).

A medical stent operated (or moved) by the medical stent operating system of the present invention can have a first expansion section, a second expansion section and a third expansion section, wherein the third expansion section is disposed between the first expansion section and the second expansion section (referring to the Taiwan Patent Application No. 110103890 above for such medical stent. However, it should be understood that, the medical stent operating system is not limited to being applicable to only such type of medical stent). The medical stent operating system of the present invention includes a staged movement mechanism, and the operating portion moves in stages relative to the holding portion via the staged movement mechanism, further sequentially pushing the first expansion section, the third expansion section and the second expansion section in stages out of the tube sheath portion by the stent movement portion. The staged movement mechanism can be, for example but not limited to, an accommodation device shown in FIG. 7 to FIG. 8C as that in the Taiwan Patent Application No. 110103890 above.

Referring to FIG. 2A, FIG. 2B and FIG. 2C, the staged movement mechanism of the medical stent operating system can be the holding portion 2200, of which the holding body 2210 including the fastening holes 2232, 2234 and 2236, and the first operating end 2110 of the operating portion 2100 includes a fastening unit 2130. During a process of the operating portion 2100 moving relative to the holding portion 2200, the fastening unit 2130 can be fastened with one of the fastening holes 2232, 2234 and 2236. When the fastening unit 2130 is fastened with the fastening hole 2236 (the fastening hole 2236 can be referred to as a first fastening hole), the first expansion section of the medical stent is pushed out of the tube sheath end 2320 of the tube sheath portion 2300 by the pushing unit 2410 of the stent movement portion 2400. When the fastening unit 2130 is fastened with the fastening hole 2234 (the fastening hole 2234 can be referred to as a second fastening hole), the third expansion section of the medical stent is pushed out of the tube sheath end 2320 of the tube sheath portion 2300 by the pushing unit 2410 of the stent movement portion 2400. When the fastening unit 2130 is fastened with the fastening hole 2232 (the fastening hole 2232 can be referred to as a third fastening hole), the second expansion section of the medical stent is pushed out of the tube sheath end 2320 of the tube sheath portion 2300 by the pushing unit 2410 of the stent movement portion 2400. With the staged movement mechanism above, a user is enabled to respectively place the first expansion section, the third expansion section and the second expansion section of the medical stent in a staged manner to correct positions in a human body (wherein correct placement positions of the first expansion section, the third expansion section and the second expansion section can be referred from the Taiwan Patent Application No. 110103890 above). Preferably, when the fastening unit 2130 is fastened with any one of the fastening holes 2232, 2234 and 2236, a user can still release the fastening between the fastening unit 2130 and the corresponding fastening hole by applying a larger force, and to enable the operating portion 2100 to be continuously pushed or pulled relative to the holding portion 2200.

The protruding unit 2140 of the operating portion 2100 can protrude outward through the elongated through hole 2240 (the configuration details thereof can be referred from the protruding unit 1140 and the elongated through hole 1240 depicted in FIG. 1), and the protruding unit 2140 is movable along the elongated through hole 2240 of the holding portion 2200. Thus, when the operating portion 2100 moves relative to the holding portion 2200, the protruding unit 2140 does not affect operations between the operating portion 2100 and the holding portion 2200. However, during a process of the fastening unit 2130 moving from the fastening hole 2234 to the fastening hole 2232 and before the fastening unit 2130 is fastened with the fastening hole 2232, the protruding unit 2140 first comes into contact with the through holed end 2242 of the elongated through hole 2240, such that the operating portion 2100 is unable to continue moving relative to the holding portion 2200 along the push-out direction 820. At this point, the second expansion section of the medical stent is partially exposed outside the tube sheath end 2320 of the tube sheath portion 2300. The mechanism above is capable of preventing the second expansion section from being completely pushed out of the tube sheath end 2320 too soon due to an excessive force applied, hence preventing the second expansion section from being placed at an incorrect position or from causing damage on the human body by the medical stent.

Once the protruding unit 2140 comes into contact with the through hole end 2242 of the elongated through hole 2240, a user can apply a first external force (for example, the first external force is a force pressing the protruding unit 214 and perpendicular to an extension direction of the operating portion 2100) on the protruding unit 2140, such that the protruding unit 2140 departs from the through hole end 2242 and departs from the elongated through hole 2240. The user can next apply a second external force on the operating portion 2100 (that is, continuing pushing the operating portion 2100 in the direction 830), such that the operating portion 2100 moves relative to the holding portion 2200 along the push-out direction 820, and the second expansion section of the medical stent is pushed out of the tube sheath end 2320 of the tube sheath portion 2300 by the pushing unit 2410 of the stent movement portion 2400.

The holding fastening unit 2250 of the holding portion 2200 can be fastened with the fastening hole 2360 of the tube sheath portion 2300, so as to more securely connect the holding portion 2200 at the tube sheath portion 2300. A user can release the fastening between the fastening unit 2250 and the fastening hole 2360 as required, further separating the holding portion 2200 from the tube sheath portion 2300. For example, during a process of a user removing the medical stent from the inside to the outside of the body of a patient by the medical stent operating system, the medical stent can be first hooked by the hooking portion of the stent movement portion 2400, then the fastening between the fastening unit 2250 and the fastening hole 2260 is released, and the stent movement portion 2400 and/or the extension unit 2220 of the holding portion 2200 are removed from the inside to the outside of the tube sheath extension unit 2330 by pulling the operating portion 2100 and/or the holding portion 2200. Thus, the medical stent is removed to the outside of the body along with the hook unit.

The medical stent operating system of the present invention has been described in detail by way of the above description and drawings. However, it should be understood that, the specific embodiments of the present invention are for illustration purposes, and various modifications that may be made without departing from the scope of claims and spirit of the present invention are to be encompassed within the scope of the appended claims. Therefore, the specific embodiments given in the description of the present disclosure are not to be construed as limitations to the present invention, and the essential scope and spirit of the present invention are as disclosed in the appended claims.

## Claims

1. A medical stent operating system, comprising:
a tube sheath portion, having a first cavity, the first cavity extending along a length direction of the tube sheath portion;
a holding portion, connected to the tube sheath portion, having a second cavity;
an operating portion, movably connected to the holding portion, the operating portion comprising a first operating end and a second operating end, the first operating end disposed in the second cavity, and the second operating end disposed outside the holding portion; and
a stent movement portion, connected to the first operating end and extending into the first cavity and into the second cavity, an end of the stent movement portion being in contact with a medical stent;
wherein the operating portion moves relative to the holding portion and moves the medical stent via the stent movement portion.

2. The medical stent operating system according to claim 1, wherein the tube sheath portion appears as a transparent tube.

3. The medical stent operating system according to claim 1, further comprising:
an optical sensing device, comprising a sensing unit and a transmission unit, the sensing unit connected to the transmission unit;
wherein the sensing unit is disposed on a holding end of the holding portion,
wherein the sensing unit is at least partially disposed in the tube sheath portion, and is located on a tube sheath end of the tube sheath portion, and
wherein the transmission unit is at least partially extendedly disposed in the first cavity.

4. The medical stent operating system according to claim 3, wherein the stent movement portion comprises a pushing unit disposed on an end of the stent movement portion, and the pushing unit is shaped as a hollow cylinder and sleeves the transmission unit in a manner of being movable relative to the transmission unit.

5. The medical stent operating system according to claim 1, wherein the tube sheath portion comprises a tube sheath end having a lateral communication outlet.

6. The medical stent operating system according to claim 1, wherein the stent movement portion comprises a hook unit disposed on an end of the stent movement portion.

7. The medical stent operating system according to claim 1, wherein the tube sheath portion is detachably connected to the holding portion.

8. The medical stent operating system according to claim 1, wherein the stent movement portion comprises a pushing unit disposed on an end of the stent movement portion.

9. The medical stent operating system according to claim 1, wherein
the medical stent has a first expansion section, a second expansion section and a third expansion section, the third expansion section disposed between the first expansion section and the second expansion section;
wherein the medical stent operating system comprises a staged movement mechanism, and the operating portion moves in stages relative to the holding portion via the staged movement mechanism, and sequentially pushes the first expansion section, the third expansion section and the second expansion section in stages out of the tube sheath portion by the stent movement portion.

10. The medical stent operating system according to claim 9, wherein the staged movement mechanism is the holding portion having a plurality of fastening holes, the first operating end of the operating portion comprises a fastening unit, and the fastening unit fastens with one of the plurality of fastening holes during a process of the operating portion moving relative to the holding portion.

11. The medical stent operating system according to claim 10, wherein
the plurality of fastening holes comprise a first fastening hole, a second fastening hole and a third fastening hole,
wherein the first expansion section is pushed out of the tube sheath portion by the stent movement portion when the fastening unit is fastened with the first fastening hole,
wherein the third expansion section is pushed out of the tube sheath portion by the stent movement portion when the fastening unit is fastened with the second fastening hole, and
wherein the second expansion section is pushed out of the tube sheath portion by the stent movement portion when the fastening unit is fastened with the third fastening hole.

12. The medical stent operating system according to claim 1, wherein the holding portion has an elongated through hole extendedly disposed on the holding portion along a length direction of the holding portion, the first operating end of the operating portion comprises a protruding unit, and the protruding unit protrudes outward through the elongated through hole and is movable along the elongated through hole.

13. The medical stent operating system according to claim 12, wherein
the medical stent has a first expansion section, a second expansion section and a third expansion section, the third expansion section disposed between the first expansion section and the second expansion section;
wherein the elongated through hole comprises a through hole end, and the medical stent operating system causes the protruding unit to come into contact with the through hole end such that the operating portion is unable to continue moving relative to the holding portion along a push-out direction; and
wherein when the protruding unit is in contact with the through hole end, the medical stent operating system causes the second expansion section of the medical stent to be partially exposed outside the tube sheath portion via the stent movement portion.

14. The medical stent operating system according to claim 13, wherein the protruding unit receives a first external force, departs from the through hole end and departs from the elongated through hole, and the operating portion next receives a second external force and moves relative to the holding portion along the push-out direction, and causes the second expansion section to be pushed out of the tube sheath portion by the stent movement portion.

15. The medical stent operating system according to claim 1, wherein the tube sheath portion comprises a liquid transport unit in communication with the first cavity, and the medical stent operating system transports a liquid into the tube sheath portion via the liquid transport unit.
